# EUROPÄISCHE PATENTSCHRIFT

(11) **EP 1 224 315 B1**
(45) Veröffentlichungstag und Bekanntmachung des Hinweises auf die Patenterteilung: **08.03.2006**
(21) Anmeldenummer: 00975925.9
(22) Anmeldetag: 26.10.2000
(51) Int. Cl.: C12P 7/24, C12P 7/40, C12N 1/21, C12R 1/19

(54) **MIKROBIOLOGISCHES VERFAHREN ZUR HERSTELLUNG AROMATISCHER ALDEHYDE UND/ODER CARBONSAUREN**
MICROBIOLOGICAL METHODS FOR THE PRODUCTION OF AROMATIC ALDEHYDES AND/OR CARBOXYLIC ACIDS
PROCEDE MICROBIOLOGIQUE DE FABRICATION D'ALDEHYDES ET/OU D'ACIDES CARBOXYLIQUES AROMATIQUES

(30) Priorität: 27.10.1999 DE 19951768
(43) Veröffentlichungstag der Anmeldung: 24.07.2002
(60) Teilanmeldung: 06000642.6
(73) Patentinhaber: BASF Aktiengesellschaft, 67056 Ludwigshafen (DE)
(72) Erfinder: SCHMID, Andreas, DE-44229 Dortmund (DE); WITHOLT, Bernard, CH-8093 Zürich (CH); HAUER, Bernhard, 67136 Fu gönheim (DE); BÜHLER, Bruno, DE-44143 Dortmund (DE)
(74) Vertreter: Kinzebach, Werner
(86) Internationale Anmeldenummer: PCT/EP2000/010552
(87) Internationale Veröffentlichungsnummer: WO 2001/031047

(56) Entgegenhaltungen:
- EP-A- 0 442 430
- EP-A- 0 466 115
- EP-A- 0 477 828
- WO-A-95/02061
- HARAYAMA S ET AL.: "Characterization of five genes in the upper-pathway operon of TOL plasmid pWWO from Pseudomonas putida and identification of the gene products" JOURNAL OF BACTERIOLOGY, vol. 171, no. 9, September 1989 (1989-09), pages 5048-5055, XP001001239 cited in the application
- PANKE S ET AL.: "An alkane-responsive expression system for the production of fine chemicals" APPLIED AND ENVIRONMENTAL MICROBIOLOGY, vol. 65, no. 6, June 1999 (1999-06), pages 2324-2332, XP002166421 cited in the application
- HARAYAMA S ET AL.: "Gene order of the TOL catabolic plasmid upper pathway operon and oxidation of both toluene and benzyl alcohol by the xylA product" JOURNAL OF BACTERIOLOGY, vol. 167, no. 2, August 1986 (1986-08), pages 455-461, XP001001260 cited in the application
- SHAW J P ET AL.: "Characterization in vitro of the hydroxylase component of xylene monooxygenase, the first enzyme of the TOL-plasmid-encoded pathway for the mineralization of toluene and xylenes" J. FERMENT. BIOENG., vol. 79, no. 3, 1995, pages 195-199, XP001001216 cited in the application
- WUBBOLTS M G ET AL.: "TOL plasmid-specified xylene oxygenase is a wide-substrate range monooxygenase capable of olefin epoxidation" ENZYME MICROB. TECHNOL., vol. 16, July 1994 (1994-07), pages 608-615, XP000990963 cited in the application
- WUBBOLTS M: "Xylene and alkane mono-oxygenases from Pseudomonas putida - genetics, regulated expression and utilizytion in the synthesis of optically active synthons" 4 March 1994 (1994-03-04) , RIJKSUNIVERSITEIT GRONINGEN , GRONINGEN XP002166423 cited in the application page 7-24
- KIENER A: "Biosynthesis of functionalized aromatic N-heterocycles" CHEMTECH, vol. 25, no. 9, 1 September 1995 (1995-09-01), pages 31-35, XP000567309
- BÜHLER B ET AL.: "Xylene monooxygenase catalyzes the multistep oxygenation of toluene and pseudomucene to corresponding alcohols, aldehydes, and acids in Escherichia coli JM101" JOURNAL OF BIOLOGICAL CHEMISTRY, vol. 275, no. 14, 7 April 2000 (2000-04-07), pages 10085-10092, XP002166422

## Beschreibung

Die Erfindung betrifft ein oxidatives mikrobiologisches Verfahren zur Herstellung aromatischer Aldehyd- und/oder Carbonsäure-Derivate unter Verwendung von Xylolmonooxygenase exprimierenden rekombinanten Mikroorganismen.

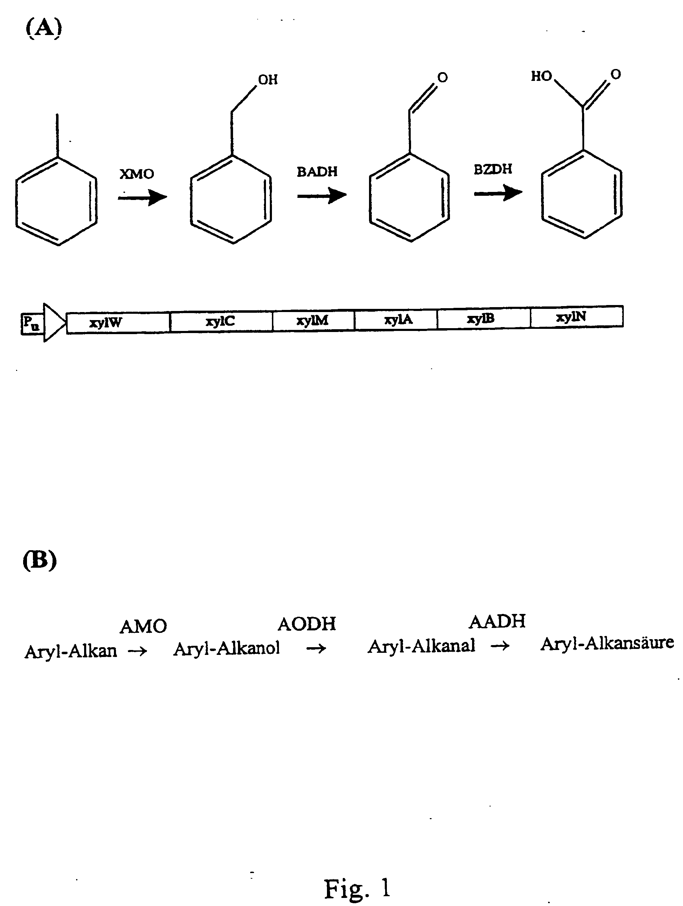

Xylolmonooxygenase (XMO), wie sie z.B. von dem TOL-Plasmid pWWO von Pseudomonas putida mt-2 kodiert wird, ist ein Enzymsystem, dem beim Abbau von Toluol und Xylolen eine Schlüsselrolle zukommt. XMO gehört zur Familie der Alkylgruppen-Hydroxylasen und hydroxyliert selektiv eine Methylgruppe am aromatischen Ring. Dies ist der erste Schritt eines Stoffwechselweges (vgl. Figur 1 (A)) der zur Bildung von Carbonsäure-Derivaten führt, die dann über den sogenannten meta-Abbauweg in Substrate für den Krebs-Zyklus umgewandelt werden.

XMO besteht aus zwei Polypeptid-Untereinheiten XylM und XylA, die von den Genen xylM und xylA kodiert werden (xylMA GENBANK-Accession-Nr. M37480) XylA ist eine NADH-Akzeptor-Reduktase, d.h. ein Elektronen-Transportprotein, das Reduktionsäquivalente von NADH auf XylM, eine in der Membran lokalisierte Hydroxylase überträgt.

Die Aktivität von XylM ist von der Gegenwart von Phospholipiden und Eisen(II)-Ionen abhängig und hat ein pH-Optimum von 7. Die Aminosäuresequenz von XylM ist zu 25% mit der Aminosäuresequenz der Hydroxylasekomponente AlkB der Alkan-Hydroxylase von *P. oleovorans* GPo1 homolog.

Die Alkanhydroxylase ist das erste Enzym eines Abbauweges für Alkane mit mittlerer Kettenlänge, an dem ein Satz von Enzymen beteiligt ist, der von zwei alk-Genclustern auf dem katabolischen OCT-Plasmid kodiert wird.

Das zweite Enzym in dem in Figur 1 (A) dargestellten Stoffwechselweg ist die Benzylalkoholdehydrogenase (BADH), ein homodimeres Mitglied einer zinkhaltigen Dehydrogenase-Familie, deren Substrate langkettige Alkohole sind. Dieses Enzym wird vom xylB-Gen kodiert. Das dritte Enzym in dem in Figur 1 (A) dargestellten Stoffwechselweg ist die Benzaldehyddehydrogenase (BZDH), bei der es sich ebenfalls um ein Homodimer handelt, das vom xylC-Gen kodiert wird.

Weitere Einzelheiten zu den Funktionen und Eigenschaften der oben erwähnten Gene und Enzyme, finden sich in den Literaturstellen (1) bis (18).

Es konnte gezeigt werden, dass *Escherichia coli,* die gentechnisch so rekombiniert wurden, dass sie XMO exprimieren, nicht nur Toluol und Xylole, sondern auch m- und p-Ethyl-, Methoxy-, Nitro- und Chlor-substituierte Toluole sowie m-Brom-substituiertes Toluol zu den entsprechenden Benzylalkohol-Derivaten oxidieren können (19, 20). Styrol wird zu Styroloxid (ee 95%) oxidiert. Darüber hinaus wurde vermutet, dass XMO auch den zweiten Schritt in dem in Figur 1 (A) dargestellten Stoffwechselweg katalysiert, nämlich die Oxidation von Benzylalkoholen zu den entsprechenden Aldehyden *in vivo* (d.h. bei Versuchen mit ganzen, lebenden Zellen) (2, 21). Auch die Umwandlung von Benzaldehyd in Benzoat nach dem dritten Schritt des in Figur 1 (A) dargestellten Stoffwechselweges konnte bereits beobachtet werden, wurde aber unspezifischen Dehydrogenasen in E. *coli* zugeschrieben (2). Weitere *in vitro* durchgeführte Untersuchungen mit teilweise gereinigter XylMA (in dieser Beschreibung auch synonym für XMO verwendet, d.h. für das aus zwei Polypeptid-Untereinheiten, nämlich XylM und XylA, bestehende funktionelle Enzym) haben dagegen gezeigt, dass diese keine Aktivität in Bezug auf Benzylalkohol hat (9). Die Gründe für diese Diskrepanz sind nach wie vor unklar.

Aufgrund der ausgeprägten Homologie zwischen Xylolmonooxygenase und Alkanmonooxygenase (AMO, auch als Alkanhydroxylase bezeichnet; GENBANK Accession-Nr.: AJ245436) hätte der Fachmann für beide Systeme ähnliche Einschränkungen hinsichtlich Art und Umfang der katalysierten Reaktionen erwartet.

Die bisher aus dem Stand der Technik bekannten biokatalytischen Verfahren zur Herstellung aromatischer Aldehyde bzw. Carbonsäuren waren insofern noch nicht zufriedenstellend, als zu deren Durchführung verschiedene Enzyme notwendig erschienen. Auch eine chemische Synthese erweist sich aufgrund der erforderlichen Regio- und Chemoselektivität als schwierig.

Es war deshalb Aufgabe der Erfindung, ein vereinfachtes Verfahren zur Herstellung aromatischer Aldehyde und/oder Carbonsäuren bereitzustellen.

Erfindungsgemäß konnte überraschenderweise ein vereinfachtes mikrobiologisches Herstellungsverfahren bereitgestellt werden. Die Erfindung beruht insbesondere auf der überraschenden Erkenntnis, dass XMO in der Lage sind, jeden einzelnen Schritt des in Figur 1 (A) bzw. 1 (B) dargestellten Reaktionsweges zu katalysieren, d.h. die Oxidation von alkylsubstituierten Aromaten zum Carbonsäure-Derivat über das entsprechende Alkohol-Derivat und das entsprechende Aldehyd-Derivat als Zwischenstufen. Überraschenderweise wurde erfindungsgemäß auch festgestellt, dass im Vergleich zu früheren Untersuchungen mit XMO exprimierenden, rekombinanten E. coli (20, 21), unter Verwendung eines speziellen Expressionssystems für die XMO-Gene, d.h. xylM und xylA, eine 10-bis 20-fach höhere Aktivität erzielbar ist.

Ein erster Gegenstand der Erfindung betrifft somit ein Verfahren zur Herstellung aromatischer Aldehyde und/oder Carbonsäuren der allgemeinen Formel I

Ar-(CH₂)ₙ-R¹ (I)

worin
- Ar: für einen gegebenenfalls ein- oder mehrfach substituierten einkernigen aromatischen Ring steht;
- R¹: für eine Sauerstoff-haltige Gruppe -CHO oder -COOH steht; und
- n: für eine ganzzahligen Wert von 0 bis 15, wie z.B.0 bis 12, 1 bis 6 oder 6 bis 12 steht,
das dadurch gekennzeichnet ist, daß man
a) in einem Kulturmedium, welches ein aromatisches Substrat der Formel II

   Ar-R² (II)

   worin
   - Ar: die oben angegebenen Bedeutungen besitzt, und
   - R²: für -CH=CH₂ oder -(CH₂)ₙ₊₁R³ steht, worin n wie oben angegeben definiert ist und R³ für H oder OH steht; oder, wenn R¹ für -COOH steht, R² auch für -(CH₂)ₙR⁴ stehen kann, worin n wie oben angegeben definiert ist und R⁴ für -CHO steht;
   enthält, einen Mikroorganismus, insbesondere aerob, kultiviert, der Xylolmonoxygenase (XMO) exprimiert; und
b) die Verbindung(en) der Formel I aus dem Kulturmedium isoliert.

Die erfindungsgemäße Reaktion kann also mit dem gleichen Enzym, ein- oder mehrstufig durchgeführt werden. Als Substrat kann der alkylierte Aromat, der korresponierende Alkohol oder der korrespondierende Aldehyd eingesetzt werden. Der Oxidationsgrad des eingesetzten Substrats kann, wie unten beschrieben, in einfacher Weise gesteuert werden.

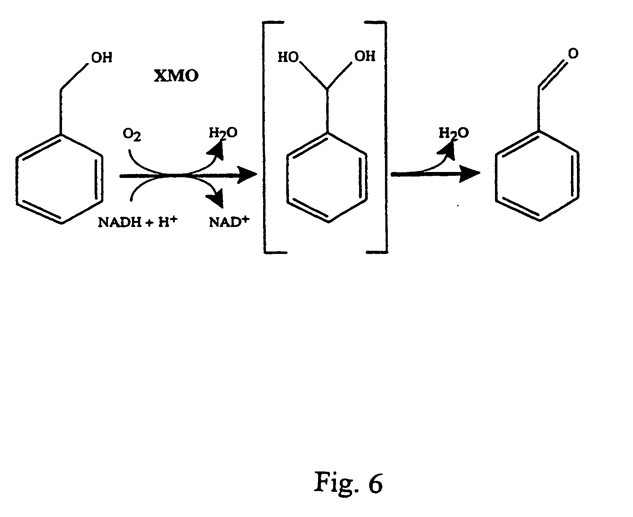

Ohne sich auf eine bestimmte Theorie festlegen zu wollen, deuten ¹⁸O-Einbauexperimente in Verbindung mit durch Massenspektrometrie erhaltenen Fragmentierungsmustern darauf hin, daß der wahrscheinlichste Mechanismus der durch XMO katalysierten Alkoholoxidation über die Bildung eines geminalen Diols als Zwischenprodukt läuft, das dann vermutlich nicht-stereospezifisch unter Erhalt des Aldehyds dehydriert wird (vgl. Fig. 6).

Das aromatische Ringsystem Ar in der erfindungsgemäß hergestellten bzw. als Substrat eingesetzten Verbindungen der Formeln I und II kann einfach oder mehrfach substituiert sein. Die Position des/der Ringsubstituenten ist beliebig wählbar. Bevorzugt ist jedoch die meta- und/oder para-Stellung zur zu oxidierenden Seitenkette.

Konkrete nichtlimitierende Beispiele für nach dem erfindungsgemäßen Verfahren durch XMO oxidierbare Substrate der Formel II sind Toluol, Xylole, Styrol, m- und/oder p- Methyl, Ethyl-, Methoxy-, Nitro- und Chlor-substituierte Toluole, sowie m-Brom-substituiertes Toluol und Pseudocumol (d.h. Trimethylbenzole); sowie die korrespondierenden Alkohole bzw. Aldehyde dieser Verbindungen. Konkrete nichtlimitierende Beispiele für nach dem erfindungsgemäßen Verfahren durch AMO oxidierbare Substrate der Formel II sind Toluol, Ethylbenzol, n- und i-Propylbenzol, n-Butylbenzol, sowie die m- und/oder p- Methyl, Ethyl-, Methoxy-, Nitro- und Chlorsubstituierten Analoga dieser Verbindungen; und die korrespondierenden Alkohole und Aldehyde dieser Verbindungen.

Die erfindungsgemäßen Verfahren werden bevorzugt unter Einsatz folgender Enzyme durchgeführt:

XMO, kodiert von den Genen xylA und xylB gemäß xylMA GENBANK-Accession Nr. M37480 und korrespondierende Isoenzyme. XMO stammt bevorzugt aus Bakterien der Gattung Pseudomonas, insbesondere der Spezies Pseudomonas putida, bevorzugt Stamm mt-2 (ATCC 33015).

Erfindungsgemäß mit umfasst ist ebenfalls die Verwendung "funktionaler Äquivalente" der konkret offenbarten XMO's und AMO's.

"Funktionale Äquivalente" oder Analoga der konkret offenbarten Monooxygenasen sind im Rahmen der vorliegenden Erfindung davon verschiedene Enzyme, welche weiterhin die gewünschte Reaktion zeigen und zur Herstellung von Aldehyden und/oder Carbonsäuren obiger allgemeiner Formel I brauchbar sind.

Unter "funktionalen Äquivalenten" versteht man erfindungsgemäß insbesondere Enzymmutanten, welche in wenigstens Sequenzposition eine andere als die ursprüngliche Aminosäure aufweisen aber trotzdem eine der oben genannten oxidationsreaktionen katalysieren. "Funktionale Äquivalente" umfassen somit die durch eine oder mehrere Aminosäure-Additionen, -Substituenten, -Deletionen und/oder -Inversionen erhältlichen Mutanten, wobei die genannten Veränderungen in jeglicher Sequenzposition auftreten können, solange sie zu einer Mutante mit dem erfindungsgemäßen katalytischen Aktivität führen. Funktionale Äquivalenz ist insbesondere auch dann gegeben, wenn die Reaktivitätsmuster zwischen Mutante und unverändertem Enzym qualitativ übereinstimmen, d.h. beispielsweise gleiche Substrate mit unterschiedlicher Geschwindigkeit umgesetzt werden.

"Funktionale Äquivalente" umfassen natürlich auch Monooxygenasen, welche aus anderen Organismen, z.B. aus anderen als den hierin konkret genannten Bakterien, zugänglich sind, sowie natürlich vorkommende Varianten oder Isoezyme. Beispielsweise lassen sich durch Sequenzvergleich Bereiche homologer Sequenzregionen festlegen und in Anlehnung an die konkreten Vorgaben der Erfindung äquivalente Enzyme ermitteln.

Erfindungsgemäß mit umfaßt ist auch die Verwendung von anderen als den konkret genannten Nukleinsäuresequenzen (einzel- und doppelsträngige DNA- und RNA-Sequenzen) welche für eine der obigen Monooxygenasen und deren funktionalen Äquivalenten kodieren. Weitere erfindungsgemäß brauchbare Nukleinsäuresequenzen unterscheiden sich somit von den konkret eingesetzten Sequenzen durch Addition, Substitution, Insertion oder Deletion einzelner oder mehrerer Nukleotide, kodieren aber weiterhin für eine Monooxygenase mit der gewünschten Eigenschaftsprofil.

Erfindungsgemäß umfasst ist auch der Einsatz solcher Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten, davon. Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind außerdem Expressionskonstrukte, enthaltend unter der genetischen Kontrolle regulativer Nukleinsäuresequenzen eine für ein erfindungsgemäß brauchbares Monooxygenase-Enzym kodierende Nukleinsäuresequenz; sowie Vektoren, umfassend wenigstens eines dieser Expressionskonstrukte. Vorzugsweise umfassen solche erfindungsgemäßen Konstrukte 5'-strom-aufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz. Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Translationsverstärker, Enhancer, Polyadenylierungssignale und dergleichen. Weitere regulative Elemente umfassen selektierbare Marker, Amplifikationssignale, Replikationsursprünge und dergleichen.

Zusätzlich zu den artifiziellen Regulationssequenzen kann die natürliche Regulationssequenz vor dem eigentlichen Strukturgen noch vorhanden sein. Durch genetische Veränderung kann diese natürliche Regulation gegebenenfalls ausgeschaltet und die Expression der Gene erhöht oder erniedrigt werden. Das Genkonstrukt kann aber auch einfacher aufgebaut sein, das heißt es werden keine zusätzlichen Regulationssignale vor das Strukturgen insertiert und der natürliche Promotor mit seiner Regulation wird nicht entfernt. Statt dessen wird die natürliche Regulationssequenz so mutiert, dass keine Regulation mehr erfolgt und die Genexpression gesteigert oder verringert wird. Die Nukleinsäuresequenzen können in einer oder mehreren Kopien im Genkonstrukt enthalten sein.

Beispiele für brauchbare Promotoren sind: cos-, tac-, trp-, tet-, trp-tet-, lpp-, lac-, lpp-lac-, lacIq-, T7-, T5-, T3-, gal-, trc-, ara-, SP6-, 1-PR- oder im 1-PL-Promotor, die vorteilhafterweise in gram-negativen Bakterien Anwendung finden; sowie die gram-positiven Promotoren amy und SPO2, die Hefepromotoren ADC1, MFa , AC, P-60, CYC1, GAPDH oder die Pflanzenpromotoren CaMV/35S, SSU, OCS, lib4, usp, STLS1, B33, not oder der Ubiquitin- oder Phaseolin-Promotor. Besonders bevorzugt ist die Verwendung induzierbarer Promotoren, wie z.B. licht- oder temperaturinduztierbare Promotoren, wie der PᵣPₗ-Promotor

Prinzipiell können alle natürlichen Promotoren mit ihren Regulationssequenzen verwendet werden. Darüber hinaus können auch synthetische Promotoren vorteilhaft verwendet werden.

Die genannten regulatorischen Sequenzen sollen die gezielte Expression der Nukleinsäuresequenzen und der Proteinexpression ermöglichen. Dies kann beispielsweise je nach wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Expression positiv beeinflussen und dadurch erhöhen oder erniedrigen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Die Herstellung einer erfindungsgemäßen Expressionskassette erfolgt durch Fusion eines geeigneten Promotors mit einer geeigneten Monooxygenase-Nukleotidsequenz sowie einem Terminator- oder Polyadenylierungssignal. Dazu verwendet man gängige Rekombinations- und Klonierungstechniken, wie sie beispielsweise in T. Maniatis et al (24) sowie in T.J. Silhavy et al. (32) und in Ausubel, F.M. et al. (33) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al. (34)) entnommen werden.

Unter Vektoren sind außer Plasmiden auch alle anderen dem Fachmann bekannten Vektoren, wie beispielsweise Phagen, Viren, wie SV40, CMV, Baculovirus und Adenovirus, Transposons, IS-Elemente, Phasmide, Cosmide, und lineare oder zirkuläre DNA zu verstehen.

Diese Vektoren können autonom im Wirtsorganismus repliziert oder chromosomal repliziert werden.

Mit Hilfe solcher erfindungsgemäßer Vektoren sind rekombinante Mikroorganismen herstellbar, welche beispielsweise mit wenigstens einem erfindungsgemäßen Vektor transformiert sind und im erfindungsgemäßen Verfahren eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al. (35) beschrieben.

Als Wirtsorganismen sind prinzipiell alle Organismen geeignet, die eine Expression der erfindungsgemäßen Nukleinsäuren, ihrer Allelvarianten, ihrer funktionellen Äquivalente oder Derivate ermöglichen und zur Durchführung der erfindungsgemäßen mikrobiologischen Oxidationsreaktion einsetzbar sind. Unter Wirtsorganismen sind beispielsweise Bakterien, Pilze, Hefen, pflanzliche oder tierische Zellen zu verstehen. Bevorzugte Organismen sind Bakterien.

Bevorzugt wird jedoch ein solcher XMO exprimierender Mikroorganismus verwendet, der im wesentlichen keine Benzylalkoholdehydrogenase (BADH) und/oder keine Benzaldehyddehydrogenase (BZDH)-Aktivität besitzt.

Weiterhin ist bevorzugt, solche AMO exprimierenden Mikroorganismen zu verwenden, die im wesentlichen keine Alkanoldehydrogenase (AODH) und/oder Alkanaldehydrogenase (AADH)-Aktivität besitzen, welche von den Genen alkJ bzw. alkH kodiert werden. Beispielsweise kann als Mikroorganismus ein Bakterium der Gattung *Escherichia,* wie z. B. *E. coli,* beispielsweise der Stamm W3110 und einer der K12-Stämme, wie JM101 und DH5α, oder einer der *Pseudomonas putida-Stämme*, wie der Stamm KT 2440 verwendet werden Die Charakteristika einiger bevorzugter *E*. *coli* Stämme sind in Tabelle I angegeben.

Die Transformation von Mikroorganismen mit einem Vektor erfolgt erfindungsgemäß nach etablierten Standardtechniken (24) und bedarf daher keiner detaillierteren Erörterung.

Die Selektion erfolgreich transformierter Organismen kann durch Markergene erfolgen, die ebenfalls im Vektor oder in der Expressionskassette enthalten sind. Beispiele für solche Markergene sind Gene für Antibiotikaresistenz und für Enzyme, die eine farbgebende Reaktion katalysieren, die ein Anfärben der transformierten Zelle bewirkt. Diese können dann mittels automatischer Zellsortierung selektiert werden. Erfolgreich mit einem Vektor transformierte Mikroorganismen, die ein entsprechendes Antibiotikaresistenzgen (z.B. G418 oder Hygromycin) tragen, lassen sich durch entsprechende Antibiotika-enthaltende Medien oder Nährböden selektieren. Markerproteine, die an der Zelloberfläche präsentiert werden, können zur Selektion mittels Affinitätschromatographie genutzt werden.

Die Kombination aus den Wirtsorganismen und den zu den Organismen passenden Vektoren, wie Plasmide, Viren oder Phagen, wie beispielsweise Plasmide mit dem RNA-Polymerase/Promoter-System, die Phagen λ oder µ oder andere temperente Phagen oder Transposons und/oder weiteren vorteilhaften regulatorischen Sequenzen bildet ein Expressionssystem.

Gemäß einer besonders bevorzugten Ausführungsform verwendet man einen rekombinanten Mikroorganismus, welcher mit einem Expressionsvektor transformiert ist, der, z.B. unter der genetischen Kontrolle des alk-Regulationssystems *aus Pseudomonas oleovorans* GPol, die für XMO kodierenden Gene xylM und xylA, oder die für AMO kodierende Gene alkB, alkG und alkT in operativer Verknüpfung enthält.

Insbesondere bevorzugt ist der Mikroorganismus mit dem xylMA kodierenden Expressionsplasmid pSPZ3 transformiert.

Das alk-Regulationssystem aus *Pseudomonas oleovorans* GPol ist an sich bekannt. Die Expression des ersten der zwei oben erwähnen alk-Gencluster steht unter der Kontrolle von alkBp, dem alk-Promotor, und beginnt in Gegenwart des funktionellen Regulationsproteins alkS, das von dem zweiten alk-Gencluster kodiert wird, und in Gegenwart eines Induktors, wie z. B. einem Alkan, beispielsweise n-Octan, oder einer mit diesen wenig verwandten Verbindungen, wie z. B. Dicyclopropylketon (DCPK) (8, 22, 23). Die Verwendung des alk-Regulationssystems in E. coli hat den Vorteil, dass keine Katabolitrepression eintritt.

In dem erfindungsgemäßen Verfahren werden bevorzugt n-Octan und DCPK als Induktoren verwendet, und zwar besonders bevorzugt in einer Menge von 0,001 bis 0,5% (V/V) im Falle von n-Octan und in einer Menge von 0,005 bis 0,05% (V/V) im Falle von DCPK. Es können natürlich auch Gemische aus n-Octan und DCPK verwendet werden. Wenn in diesen Konzentrationsbereichen gearbeitet wird, ist die Induktion maximal.

Die Erfindung betrifft außerdem ein mikrobiologisches Verfahren zur Oxidation organischer Verbindungen obigen Typs mit Hilfe der eben beschriebenen rekombinanten Mikroorganismen. Der erfindungsgemäß eingesetzte rekombinante Mikroorganismus kann nach bekannten Verfahren kultiviert und fermentiert werden. Bakterien können beispielsweise in TB- oder LB-Medium und bei einer Temperatur von 20 bis 40°C und einem pH-Wert von 6 bis 9 vermehrt werden. Im Einzelnen werden geeignete Kultivierungsbedingungen beispielsweise in T. Maniatis et al., a.a.O, beschrieben.

Bei der erfindungsgemäßen mikrobiologischen Oxidation unter Verwendung oben beschriebener rekombinanter Mikroorganismen erfolgt vorzugsweise zunächst die Kultivierung der Mikroorganismen in Gegenwart von Sauerstoff und in einem Komplexmedium, wie z.B. TB-oder LB- Medium, bei einer Kultivierungstemperatur von etwa 20 bis 40oC oder mehr, und einem pH-Wert von etwa 6 bis 9, bis eine ausreichende Zelldichte erreicht ist. Um die Oxidationsreaktion besser steuern zu können, bevorzugt man die Verwendung eines induzierbaren Promotors. Die Kultivierung wird nach Induktion der Monooxygenaseproduktion in Gegenwart von Sauerstoff, z.B. 1 Stunde bis 3 Tage, fortgesetzt. Das gebildete Oxidationsprodukt oder -produktgemisch kann dann in herkömmlicher Weise, wie z.B. durch Extraktion oder Chromatographie, vom Medium abgetrennt und gereinigt werden.

Die erfindungsgemäßen Umsetzungen können vorteilhaft auch in Bioreaktoren durchgeführt werden, welche den erfindungsgemäßen rekombinanten Mikroorganismus, z.B. in immobilisierter Form, enthalten.

Der Oxidationsgrad der erfindungsgemäß eingesetzten Substrate kann auf einfache Weise gesteuert werden. Beispielsweise werden in regelmäßigen Abständen Proben aus dem Kulturmedium entnommen und gaschromatographisch oder unter Anwendung der Gaschromatographie-Massenspektrometer-Kopplung (GC-MS) oder der Hochleistungsflüssigkeitschromatographie auf den Gehalt an den entsprechenden Alkohol-, Aldehyd- und/oder Carbonsäure-Derivaten untersucht. Je nachdem, welches oxidierte Derivat erwünscht ist, oder wenn sich ein gewünschtes Mischungsverhältnis eingestellt hat, wird die Inkubation unterbrochen. Dies kann beispielsweise durch Entfernen oder Abtöten der Mikroorganismen aus dem Kulturmedium erfolgen, beispielsweise durch Abzentrifugieren und Dekantieren und/oder durch Behandlung mit Säure, beispielsweise Trichloressigsäure, oder durch Behandlung mit Hitze. Auch durch Zudosieren von unoxidiertem Substrat (wie z.B. Toluol oder Pseudocumol) kann die Säurebildung inhibiert werden.

Der oxidierte Aromat kann dann mit Hilfe üblicher Trennverfahren aus dem Kulturmedium isoliert werden, beispielsweise durch einfache Destillation, fraktionierte Destillation, Rektifikation, gegebenenfalls im Vakuum, oder durch Anwendung geeigneter chromatographischer Verfahren, bevorzugt durch Destillation. Zweckmäßigerweise werden die Mikroorganismus-Zellen vor der Aufreinigung dieser Produkte aus dem Kulturmedium entfernt.

Die Konstruktion des erfindungsgemäß bevorzugten Expressionsvektors pSPZ3, ist beschrieben in (31) Panke, et al., Applied and Environmental Microbiology (1999) 2324-2332, worauf hiermit ausdrücklich Bezug genommen wird.

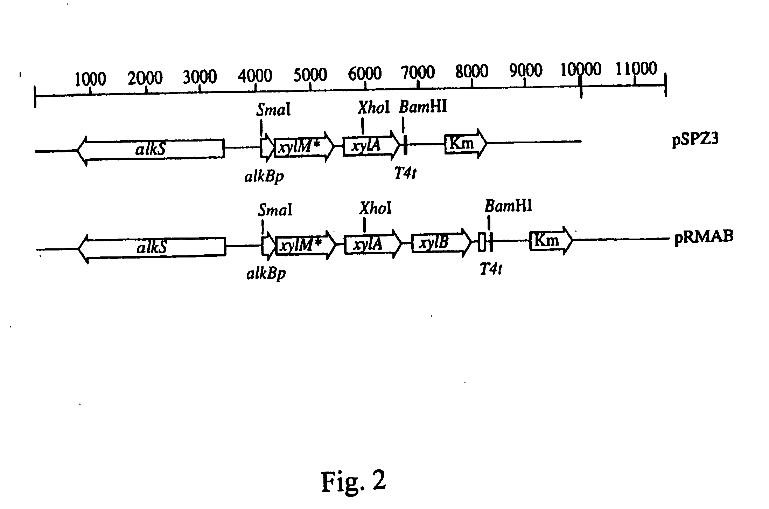

Im Rahmen der erfindungsgemäßen Untersuchungen wurde zur Transformation der Mikroorganismen auch ein Vektor verwendet, der neben den XMO-Genen xylM und xylA zusätzlich auch noch das Benzylalkoholdehydrogenase(BADH)-Gen xylB aus *Pseudomonas putida* mt-2 in exprimierbarer Form enthält. Um das Benzylalkohol-Dehydrogenase-Gen xylB direkt stromabwärts zum xylA-Gen in das oben beschriebene Plasmid pSPZ3 einzuführen, wurde das 2,3 kb lange XhoI/FspI-Fragment des Plasmids pCK04 (25), welches das xylB-Gen enthält, zuerst in den mit XhoI und SmaI verdauten Vektor pGEM-7Zf(+) (Promega, Zürich, Schweiz) unter Erhalt von pGEMAB eingeführt. Aus diesem Konstrukt wurde das 2,3 kb lange Fragment mit XhoI und BamHI ausgeschnitten und in das mit XhoI und BamHI verdaute Plasmid pSPZ3 ligiert. Das sich ergebende Plasmid wurde pRMAB genannt (Figur 2).

Die Versuche mit pRMAB haben aber gezeigt, dass in Gegenwart von BADH neben XMO Benzylalkohol nicht nur mit geringerer Aktivität zu Benzaldehyd oxidiert wird, sondern sogar Benzylalkohol rückgebildet wird.

Als negative Kontrolle wurde im Rahmen der erfindungsgemäßen Untersuchungen das Plasmid pRS, das keine xyl-Gene enthielt, konstruiert. pRMAB wurde mit BamHI und SmaI verdaut und mit Klenow-Enzym behandelt. Nach der Isolation des größeren Fragments wurde der Vektor religiert.

Im folgenden wird das erfindungsgemäße Verfahren unter Bezugnahme auf konkrete, nicht limitierende Beispiele und die Figuren erläutert.

Figur 1 zeigt (A) die stufenweise Oxidation von Toluol zu Benzylalkohol, Benzaldehyd und Benzoesäure durch die Enzyme des oberen TOL-Stoffwechselweges und die Organisation der xyl-Gene des oberen TOL-Operons. BADH und BZDH stehen für Benzylalkohol-Dehydrogenase bzw. Benzaldehyd-Dehydrogenase. Pᵤ bezeichnet den oberen TOL-Operon-Promoter, xylw ein Gen mit unbekannter Funktion, xylC das BZDH-kodierende Gen, xylM das die terminale Hydroxylase-Komponente von XMO kodierende Gen, xylA das die NADH:Akzeptor-Reduktase-Komponente von XMO kodierende Gen, xylB das BADH kodierende Gen und xylN ein Gen mit unbekannter Funktion; (B) die stufenweise Oxidation eines Aryl-substituierten Alkans über das korrespondierende Alkanol und Alkanal zur Alkancarbonsäure, katalysiert durch die Enzyme Alkanhydroxylase (AMO), Alkanoldehydrogenase (AODH) und Alkanaldehydrogenase (AADH).

Figur 2 zeigt Konstruktionsschemata der Expressionsplasmide pSPZ3 und pRMAB mit den Genen xylMA und xylMAB unter der Kontrolle des alk-Regulationssystems. alkBp bedeutet den Promotor des alk-Operons, alkS ist das Gen für den positiven Regulator AlkS. Die Gene xylM* und xylA kodieren die Xylol-Monooxygenase (das * bedeutet, daß im xylM-Gen eine NdeI-Stelle entfernt worden ist). Das xylB-Gen kodiert BADH. Km bezeichnet das Gen für die Kanamycin-Resistenz und T4t ist der Transkriptionsterminator des Phagen T4.

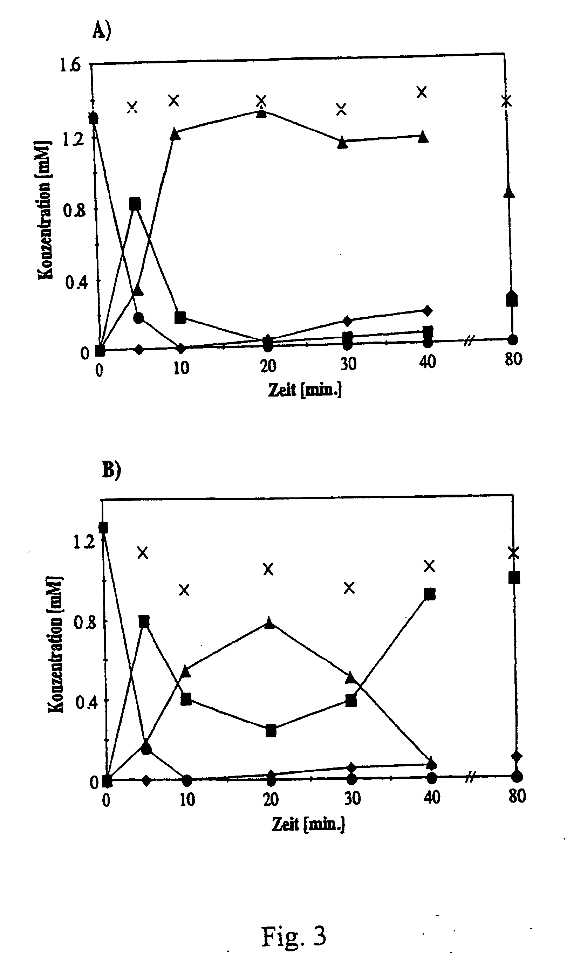

Figur 3 zeigt die Oxidation von Toluol durch *E*. *coli* JM101 (pSPZ3) (A) und *E. coli* JM101 (pRMAB) (B). Toluol (1.37 mM) wurde zu einer Suspension von ruhenden E. coli JMIOI (pSPZ3/pBRMAB) Zellen (2.07-2.14 g*l⁻¹ CDW) in Kaliumphosphatpuffer (50mM) pH 7.4, 1% (w/v) Glucose gegeben. Kreise: Toluol, Quadrate: Benzylalkohol, Dreiecke: Benzaldehyd, Rauten: Benzoesäure, Kreuze: Summe der vier Konzentrationen.

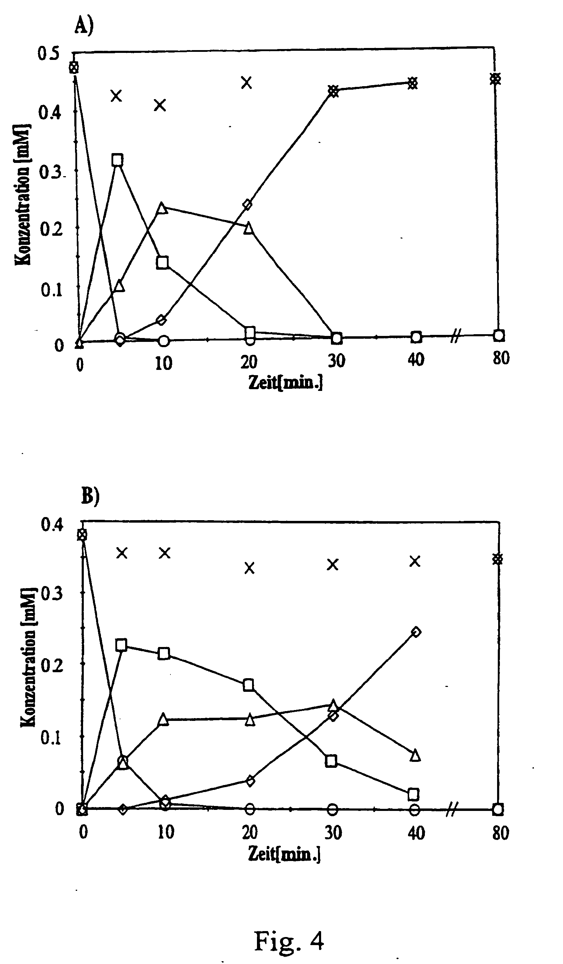

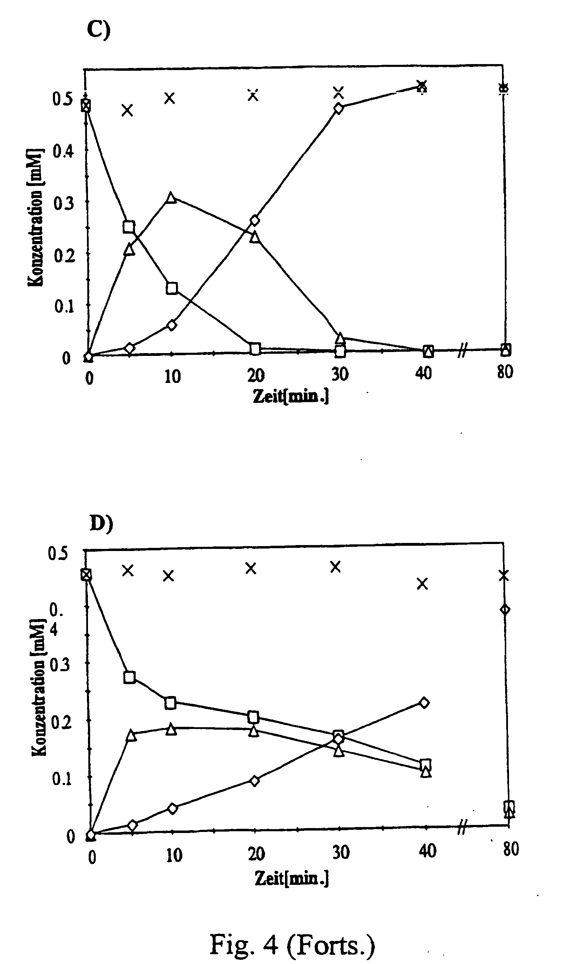

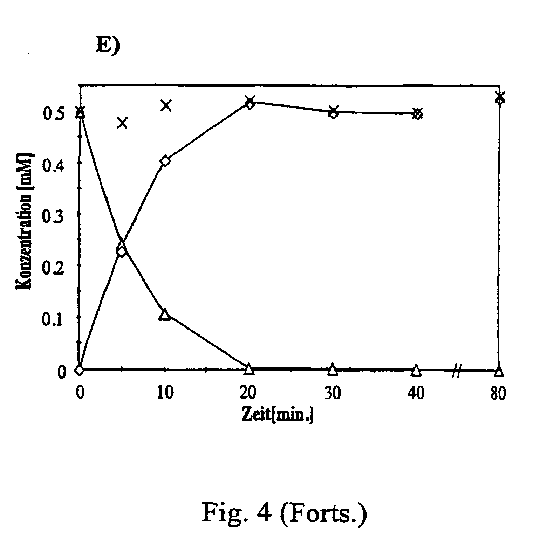

Figur 4 zeigt die Oxidation von Pseudocumol, dem entsprechenden Alkohol und dem entsprechenden Aldehyd durch *E*. *coli* JM101 (pSPZ3) (A,C,E) and *E*. *coli* JM101 (pRMAB) (B,D). Die Substrate (0.46 mM) wurden zu einer Suspension von ruhenden E. coli JM101 (pSPZ3/pBRMAB) Zellen (0.86-0.92 g*1⁻¹ CDW) in Kaliumphosphatpuffer (50mM) pH 7.4, 1 % (w/v) Glucose gegeben. Die Graphen (A) und (B) zeigen die Oxidation von Pseudocumol, die Graphen (C) und (D) die Oxidation von 3,4-Dimethylbenzylalkohol und der Graph (E) zeigt die Oxidation von 3,4-Dimethylbenzaldehyd. Kreise: Pseudocumol, Quadrate: 3,4-Dimethylbenzylalkohol, Dreiecke: 3,4-Dimethylbenzalde-hyd, Rauten: 3,4-Dimethylbenzoesäure, Kreuze: Summe aller Konzentrationen.

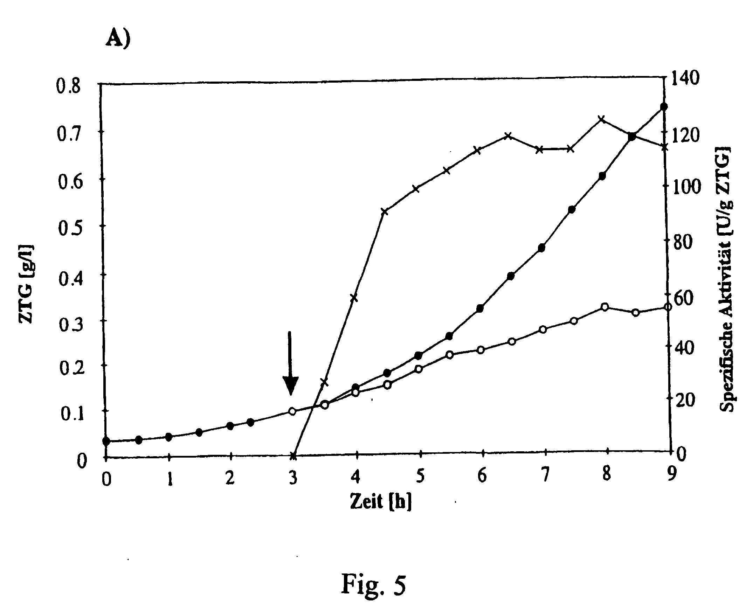

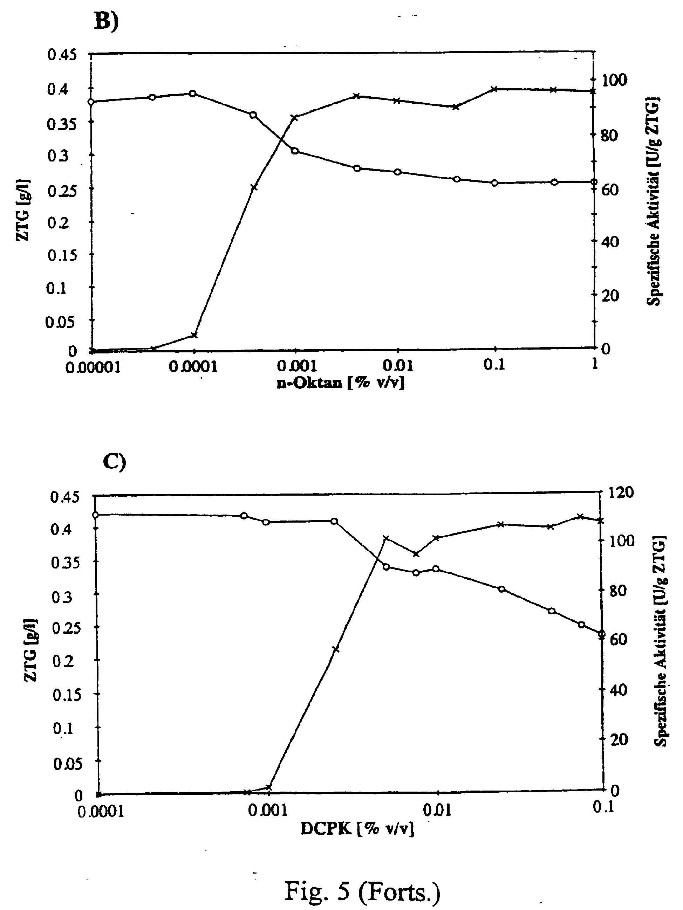

Figur 5 zeigt die Wachstums- und Induktionskinetiken von XMO in *E. coli* JM101 (pSPZ3). Graph (A) zeigt die Xylolmonooxygenaseaktivität und das Zelltrockengewicht (ZTG) zu unterschiedlichen Zeitpunkten nach Induktion durch n-Oktan während die Graphen (B) und (C) dieselben Werte 3.5 Stunden nach Induktion mit unterschiedlichen Mengen an n-Oktan respektive an Dicyclopropylketon (DCPK) zeigen. Jede Aktivitätsbestimmung stammt aus einer unabhängigen Kultur. Pseudocumol (1.37 mM) wurde zu einer Suspension von ruhenden E. coli JM101 (pSPZ3) Zellen (2.04-2.44 g*1⁻¹ CDW) in Kaliumphosphatpuffer (50mM) pH 7.4, 1 % (w/v) Glucose gegeben. Die spezifischen Aktivitäten basieren auf der Produktbildung während den ersten 5 Minuten der Reaktion. Der Pfeil in Graph (A) zeigt an, wann 0. 1 % (v/v) n-Oktan zugegeben wurde, um die XylMA Synthese zu induzieren. Ausgefüllte Kreise: ZTG von nicht induzierten Kulturen, offene Kreise: ZTG induzierter Kulturen, Kreuze: spezifische Aktivitäten induzierter Kulturen.

Figur 6 zeigt eine mögliche mechanistische Erklärung für die XMOkatalytische Bildung von Benzaldehyd aus Benzylalkohol.

### Allgemeine Methoden und verwendete Materialien

### a) Bakterien und Plasmide:

**TABELLE I Bakterienstämme und Plasmide**

| Stamm oder Plasmid | Eigenschaften | Quelle oder Literaturstelle |
|---|---|---|
| Stämme | | |
| E. coli | | |
| DH5α | supE44 ΔlacU169 (∅801acZΔM15) hsdR17 recAl endAl gyrA96 thi-1 relAl | (30) |
| JM101 | supE thi-1 Δ(lac-proAB) F'[traD26 pro AB⁺ lacI^{q} lacZΔM15] | (24) |
| Plasmide | | |
| pSPZ3 | alkS Palk xylMA ori pMB1; Km^{f} | Erfindung |
| pRMAB | pBRMA; xylB; Km^{f} | Erfindung |
| pRS | pBRMA; ΔxylMA | Erfindung |
| pCK04 | lacZa Pu xylWCMABN; pSC101 oriv; Cm^{f} | (25) |
| pGEM7Zf(+) | ColEI f1 ori lacZa; Ap^{f} | Promega |
| pGEMAB | pGEM7Zf(+); xylA*B; Ap^{f} | Erfindung |

| | | |
|---|---|---|
| A*: das Plasmid enthält lediglich einen Teil des xylA-Gens | | |

### b) Chemikalien und Enzyme

Sämtliche verwendeten Chemikalien und Enzyme sind im Handel erhältlich, beispielsweise von Boehringer Mannheim (Rotkreuz, Schweiz), NEB (Schwalbach, Deutschland), Gibco (Basel, Schweiz), AGS (Heidelberg, Deutschland), Promega (Zürich, Schweiz), Fluka (Buchs, Schweiz), Aldrich (Buchs, Schweiz) und Lancaster (Mühlheim, Deutschland). Zur Präparation von Plasmid-DNA in kleinem Maßstab wurde der QIAprep-Spin-Miniprep-Kit von Quiagen (Basel, Schweiz) nach den Angaben des Herstellers angewendet.

### c) Gentechnische Methoden

Zur Konstruktion der verwendeten Vektoren/Plasmide und zur Transfektion bzw. Transformation der Bakterien wurden Standardverfahren der Gentechnik angewendet, die beispielsweise in dem Buch von Sambrook, Fritsch und Maniatis (24) ausführlich beschrieben sind. Des weiteren wird auf die Abschnitte Material und Methoden in den im Literaturverzeichnis aufgeführten Originalarbeiten verwiesen. Eine weitergehende Erörterung ist daher entbehrlich.

### d) Aufzucht der Bakterien

Die Bakterien wurden entweder in Luria-Bertani(LB)-Brühe (Difco, Detroit, Mich.) oder in M9-Minimalmedium (24), das die dreifache Konzentration an Phosphatsalzen (M9*) und 0,5% (G/V) Glucose als einzige Kohlenstoffquelle enthielt, angezogen. Die Kulturen wurden gegebenenfalls mit Kanamycin (Endkonzentration: 50 mg/Liter), Ampicillin (100 mg/Liter), Chloramphenicol (30 mg/Liter), Thiamin (10⁻³%, G/V), 1 mM Indol und 0,5 mM IPTG (Isopropyl-β-D-1-thiogalactopyranosid) ergänzt. Feste Medien enthielten 1,5% (G/V) Agar. Flüssige Kulturen wurden routinemäßig bei 30 oder 37°C auf Horizontalschüttlern mit 200 UpM angezogen.

### e) Bestimmung der Enzymaktivität

Die Bestimmung der Enzymaktivitäten erfolgte der Einfachheit halber unter Verwendung von ganzen Zellen.

Eine Einheit (U) ist als die Aktivität definiert, die 1 µmol Gesamtprodukte in 1 Minute ergibt. Die spezifische Aktivität wird hier als Aktivität pro g Zelltrockengewicht (CDW) (U g⁻¹ CDW) ausgedrückt (im folgenden auch einfach als Aktivität bezeichnet). Berechnung als Durchschnittsaktivität, bezogen auf die Menge an Produken pro g CDW, die in den ersten 5 min der Umsetzung gebildet werden. Die Versuche wurden mindestens dreimal unabhängig voneinander wiederholt.

Der Assay wurde folgendermaßen durchgeführt. Mit den entsprechenden Vektoren rekombinierte E. coli JM101 wurden in 40 oder 100 ml Medium in Gegenwart von-Kanamycin inkubiert. Wenn die optische Dichte bei 450 nm ca. 0,3 betrug, wurden die Zellen durch Zugabe von 0,05% (V/V) DCPK- oder 0,1% (V/V) n-Octan induziert und 3 bis 3,5 Stunden weiter inkubiert, bis die OD₄₅₀ typischerweise auf 0,8-0,9 angestiegen war. Dann wurden die Zellen geerntet und bis zu einem Zelltrockengewicht von 2,5 g/l in 50 mM Kaliumphosphat-Puffer, pH 7,4, der 1% (G/V) Glucose enthielt, resuspendiert. Aliquots zu 1 oder 2 ml wurden in mit Stopfen verschlossene Pyrex-Röhrchen gegeben und horizontal auf einem Rotationsschüttler bei 30°C und 250 UpM inkubiert. Nach 5 Minuten wurde das jeweilige Substrat auf eine Endkonzentration von 1,5 mM in Form einer 20-fach konzentrierten Stammlösung in Ethanol hinzugegeben. Bei den Versuchen, in denen die Bildung von 3,4-Dimethylbenzoesäure bestimmt wurde, wurde das Zelltrockengewicht auf 1 g/l verringert und die jeweiligen Substrate bis zu einer Endkonzentration von 0,5 mM hinzugegeben, weil diese Verbindung in Wasser eine geringe Löslichkeit hat. Die Umsetzung wurde auf dem Schüttler 5 Minuten durchgeführt und dann beendet, indem die Proben in Eis gestellt und sofort mit 40 oder 80 µl Perchlorsäure-Stammlösung (10% V/V) versetzt wurden, so dass der pH-Wert der Suspension 2 betrug.

### f) Bestimmung der Produktbildung als Funktion der zeit

Zur Untersuchung der Produktbildung als Funktion der Zeit wurden die Zellen wie oben beschrieben gezüchtet, induziert, gesammelt, resuspendiert und unterschiedlich lange, nämlich 5, 10, 20, 30, 40 und 80 Minuten, mit dem jeweiligen Substrat inkubiert. Dann wurden die Umsetzungen wie oben beschrieben abgebrochen, die Zellen durch Zentrifugation (7 800 g, 8 min) entfernt und die Überstände analysiert.

Zur Auftrennung von Benzylalkohol, Benzylaldehyd und Benzoesäure wurde die Hochleistungsflüssigkeitschromatographie (HPLC) angewendet. Als Säule diente Nucleosil C18 (Porengröße 100 Å, Teilchengröße 5 µm, Länge 25 cm, Innendurchmesser 4 mm) (Macherey-Nagel, Oensingen, Schweiz) und als mobile Phase 69,9% H₂O-30% Acetonitril-0,1% H₃PO₄ bei einer Fließgeschwindigkeit von 0,7 ml/min. Zur Auftrennung von 3,4-Dimethylbenzylalkohol, 3,4-Dimethylbenzaldehyd und 3,4-Dimethylbenzoesäure wurde die gleiche Säule, aber als mobile Phase 64,9% H₂O-35% Acetonitril-0,1% H₃PO₄ mit der gleichen Fließgeschwindigkeit verwendet. Zur Detektion wurde die UV-Absorption bei 210 nm bestimmt. Die aufgetrennten Verbindungen wurden durch Vergleich ihrer Retentionszeiten mit denen von im Handel erhältlichen Standards identifiziert.

Im Falle von Toluol/Pseudocumol und der entsprechenden Alkohole, Aldehyde und Säuren erfolgte die Auftrennung durch Gaschromatographie. Der Gaschromatograph (Fisons Instruments, England) war mit einer Quarzkapillarsäule des Typs OPTIMA-5 (Länge 25 m, Innendurchmesser 0,32 mm, Filmdicke 0,25 µm) von Macherey-Nagel (Oensingen, Schweiz) ausgerüstet. Als Trägergas wurde Wasserstoff verwendet, und die Injektion erfolgte splitlos. Dabei wurde das folgende Temperaturprofil angewendet: von 40°C auf 70°C mit 15°C/min, von 70°C auf 105°C mit 5°C/min und von 105°C auf 240°C mit 20°C/min. Die Detektion der Verbindungen erfolgte mit einem Flammenionisationsdetektor. Die aufgetrennten Verbindungen wurden durch Vergleich ihrer Retentionszeiten mit denen von im Handel erhältlichen Standards identifiziert. Alternativ kann die Detektion auch mit einem Massenspektrometer erfolgen (GC-MS-Kopplung). Letzteres hat den Vorteil, dass neben der spezifischen chromatographischen Retentionszeit auch noch das Fragmentierungsmuster und die Intensitätenverteilung der einzelnen Peaks zur qualitativen und quantitativen Bestimmung der Reaktionsprodukte herangezogen werden können.

Die GC-MS-Kopplung bestand aus einem Massenspektrometer von Fisons Typ MD-800 und einem Gaschromatographen (Fisons Instruments, England), der mit einer CP-Sil-5CB-Säule (Chrompack, Niederlande) ausgerüstet war. Als Trägergas wurde Helium verwendet. Die Injektion erfolgte mit Split (20:1). Das Temperaturprogramm war das gleiche wie bei der oben beschriebenen gaschromatographischen Auftrennung.

Sowohl bei der Gaschromatographie als auch bei GC-MS-Kopplung wurden den Proben ein gleiches Volumen eiskalter Ether, der als internen Standard 0,1 mM Dodecan enthielt, zugesetzt. Anschließend wurde Natriumchlorid bis zur Sättigung hinzugegeben und die Wasserphase bei 30°C durch 5-minütiges kräftiges Schütteln extrahiert, worauf die Phasen durch Zentrifugation getrennt wurden. Die organische Phase wurde über wasserfreiem Natriumsulfat getrocknet und dann analysiert.

### Beispiel 1: Oxidation von Toluol und Derivaten davon mit Xylolmonooxygenase

In den folgenden Versuchen wurden die Zellen jeweils bis zu einer Zelldichte von 0,09 g CDW/l angezogen und routinemäßig mit 0,1% (V/V) n-Octan induziert. Dann wurden die Kulturen weitere 3 bis 3,5 Stunden inkubiert und bis auf eine Zelldichte von 0,23 bis 0,27 g CDW/1 angezogen.

Tabelle II zeigt, dass XMO Toluol zu Benzylalkohol, Benzylalkohol zu Benzaldehyd und Benzyldehyd zu Benzoesäure oxidiert. Für die ersten zwei Oxidationsreaktionen wurden Aktivitäten bis zu 95-100 U/g CDW festgestellt, wohingegen die Oxidation von Benzaldehyd mit einer niedrigen Aktivität erfolgte, nämlich nur 10 U/g CDW.

Im Fall von Pseudocumol waren die Ergebnisse ähnlich. Pseudocumol wurde zu 3,4-Dimethylbenzylalkohol, 3,4-Dimethylbenzylalkohol zu 3,4-Dimethylbenzaldehyd und 3,4-Dimethylbenzaldehyd zu 3,4-Dimethylbenzoesäure oxidiert. Für die Oxidation von Pseudocumol wurde eine Aktivität von 100 U/g CDW festgestellt, wohingegen 3,4-Dimethylbenzaldehyd langsamer mit einer Aktivität von 50 U/g CDW gebildet und mit einer deutlich höheren Aktivität (55 U/g CDW) als Benzaldehyd zu 3,4-Dimethylbenzoesäure oxidiert wurde.

Wenn die Säuren als Substrate zugegeben wurden, konnten keine Reaktionsprodukte und auch keine Abnahme der Säuren nachgewiesen werden.

Als Kontrollen dienten uninduzierte *E*. *coli* JM101, die das Plasmid pSPZ3 enthielten, und induzierte *E*. *coli* JM101, die kein Plasmid enthielten. Um jegliche Einflüsse des alk-Regulationssystems auf *E*. *coli* auszuschließen, wurden als zusätzliche Kontrollen *E. coli* JM101, die das Plasmid pRS enthielten, verwendet. Das Plasmid pRS enthält immer noch das alkS-Gen, aber nicht die xyl-Gene. Tabelle II zeigt, dass bei der Verwendung von Toluol, Pseudocumol und der entsprechenden Alkohole als Substrate keine Umwandlungsprodukte in den Kontrollversuchen nachweisbar waren.

Die folgende Tabelle III zeigt, dass trotzdem in diesem Versuch als Substrate zugegebene Aldehyde mit konstanter Aktivität zu den Alkoholen reduziert wurden. Auch die Bildung von 3,4-Dimethylbenzoesäure konnte festgestellt werden, aber mit einer sehr geringen Aktivität von 2 bis 2,5 U/g CDW. Dies erlaubt den Schluß, dass der Hauptteil der von induzierten E. coli JM101 (pSPZ3) (diese Abkürzung bedeutet hier, dass die Mikroorganismen das angegebene Plasmid enthalten) gebildeten Säure auf das Vorhandensein von XMO zurückzuführen ist.

An dieser Stelle sei darauf hingewiesen, dass in Bezug auf die gebildeten Produkte und die Aktivitäten bzw. Bildungsgeschwindigkeiten zwischen dem routinemäßig verwendeten Induktor 0,1% (V/V) n-Octan und dem alternativ verwendeten Induktor 0,05% (V/V) DCPK keine signifikanten Unterschiede festgestellt wurden.

**TABELLE II Oxidation von Toluol und Derivaten durch XMO**

| Substrat | Spezifische Aktivität^{a} [U g⁻¹ CDW] | | |
|---|---|---|---|
| | E. coli JM101 (pSPZ3) induziert^{b} | E. coli JM101 (pSPZ3) uninduziert | E. coli JM101 (pRS) induziert^{b} |
| Toluol | 100 | 0 | 0 |
| Benzylalkohol | 95 | 0 | 0 |
| Benzaldehyd | 10 | s.b.^{c} | s.b.^{c} |
| Pseudocumol | 100 | 0 | 0 |
| 3,4-Dimethylbenzyl alkohol | 50 | 0 | 0 |
| 3,4-Dimethylbenzaldehyd | 55 | s.b.^{c} | s.b.^{c} |

| | | | |
|---|---|---|---|
| a Der Assay zur Bestimmung der Aktivitat wurae wie open oeschrieben durchgeführt. Die Einheit (U) ist wie oben definiert, und die spezifische Aktivität wurde wie oben beschrieben berechnet. | | | |
| b Die Zellen wurden durch Zugabe von 0,1% (V/V) n-Octan induziert. | | | |
| c s.b., siehe unten (Tabelle III) | | | |

**TABELLE III Umwandlung von Aldehyden in den Kontrollversuchen**

| | Spezifische Aktivität^{a} [U g⁻¹ CDW] | | | |
|---|---|---|---|---|
| | Benzaldehyd als Substrat | | 3,4-Dimethylbenzaldehyd als Substrat | |
| Art des gebildeten Produktes | E. coli JM101 (pSPZ3) Uninduziert | E. coli JM101 (pRS) induziert^{b} | E. coli JM101 (pSPZ3) uninduziert | E. coli JM101 (pRS) induziert^{b} |
| Alkoholbildung | 19 | 15 | 9,4 | 8,3 |
| Säurebildung | 0,5 | 0 | 2,2 | 2,4 |

| | | | | |
|---|---|---|---|---|
| a, b vgl. Tabelle 11 | | | | |

Die in diesen Kontrollversuchen unter Verwendung von uninduzierten E. coli JM101 (pSPZ3) und induzierten E. coli JM101 (pRS) festgestellte Reduktion der Aldehyde zu den Alkoholen kann durch die Einwirkung von E.-coli-Alkohol-Dehydrogenasen erklärt werden, die eine Reaktion katalysieren, deren Gleichgewicht aus thermodynamischen Gründen auf der Seite der Alkohole liegt. Die Rückbildung von Benzylalkohol am Ende der Biotransformation von Toluol durch E. coli JM101 (pSPZ3) (vgl. Fig. 5A) kann ebenfalls den E.-coli-Dehydrogenasen zugeordnet werden, deren Aktivität signifikant wird, weil die XMO-Aktivität mit der Zeit abnimmt.

### Beispiel 2: Bestimmung des zeitlichen Verlaufs der Oxidation verschiedener Substrate

Der zeitliche Verlauf der Oxidation von Toluol, Pseudocumol, 3,4-Dimethylbenzylalkohol und 3,4-Dimethylbenzaldehyd ist in den Figuren 3 und4 dargstellt. Die Assays wurden wie oben beschrieben durchgeführt. Das jeweilige Substrat wurde jeweils zu einer Suspension der jeweiligen ruhenden Zellen in 50 mM Kaliumphosphat-Puffer, pH 7,4, der 1% (G/V) Glucose enthielt, gegeben.

Wenn Toluol oder Pseudocumol als Substrate zugegeben wurden, wurde eine aufeinanderfolgende Bildung der entsprechenden Alkohole, Aldehyde und Säuren festgestellt (Figuren 3A und 4A). Wie auch in den Aktivitätsassays festgestellt, waren die Aktivitäten, mit denen Benzylalkohol, 3,4-Dimethylbenzylalkohol und Benzaldehyd gebildet wurden, hoch. Die Aktivitäten, mit denen 3,4-Dimethylbenzaldehyd und 3,4-Dimethylbenzoesäure gebildet wurden, waren mittelhoch, und Benzoesäure bildete sich mit geringer Aktivität. In den ersten 5 Minuten wurden aus den beiden Substraten Pseudocumol und Toluol mit einer spezifischen Aktivität von 100 U/g CDW (vgl. auch Tabelle II) die Produkte gebildet. Im Bereich zwischen der 5. und 10. Minuten wurde Benzaldehyd mit einer Aktivität von 80 U/g CDW gebildet, während 3,4-Dimethylbenzaldehyd langsamer gebildet wurde (37 U/g CDW). Die Säurebildung begann nach dem vollständigen Verbrauch von Toluol oder Pseudocumol, und zwar mit einer Aktivität von 3,2 U/g bzw. 21 U/g CDW im Falle von Benzoesäure bzw. 3,4-Dimethylbenzoesäure im Bereich zwischen 10 und 30 Minuten. Es blieb stets eine geringe Konzentration an Benzylalkohol übrig. Zwischen 40 und 80 Minuten nahm die Konzentration an Benzylalkohol wieder zu, während die Konzentration an Benzaldehyd abnahm (Figur 3A). Pseudocumol, 3,4-Dimethylbenzylalkohol und 3,4-Dimethylbenzaldehyd wurden vollständig unter Bildung von 3,4-Dimethylbenzoesäure verbraucht (Figur 4A).

Wenn 3,4-Dimethylbenzylalkohol als Substrat zugegeben wurde, betrug die Aktivität, mit der 3,4-Dimethylbenzaldehyd gebildet wurde, 50 U/g CDW (Tabelle II), wohingegen die Aktivität, mit der 3,4-Dimethylbenzoesäure gebildet wurde, 23 U/g CDW betrug, und zwar im Zeitraum zwischen 10 und 30 Minuten (Figur 4C). Wenn Benzylalkohol als Substrat verwendet wurde, betrug die Aktivität, mit der Benzaldehyd gebildet wurde, 95 U/g CDW und die Aktivität, mit der Benzoesäure gebildet wurde, konstant 2,9 U/g CDW (Ergebnisse nicht gezeigt). In dem untersuchten Zeitraum wurde 3,4-Dimethylbenzylalkohol im Gegensatz zu Benzylalkohol vollständig in die Säure umgewandelt.

Wenn 3,4-Dimethylbenzaldehyd als Substrat zugegeben wurde (Figur 4E) wurde 3,4-Dimethylbenzoesäure mit einer Aktivität von 55 U/g CDW gebildet und war nach 20 Minuten bereits die vorherrschende Spezies. Wenn Benzaldehyd als Substrat verwendet wurde, wurde die langsame und konstante Bildung von Benzoesäure (3 U/g CDW) festgestellt (Ergebnisse nicht gezeigt). Hier betrug die Anfangsaktivität in den ersten 5 Minuten 10 U/g CDW (Tabelle II).

### Beispiel 3: Umwandlung von Toluol und Pseudocumol durch E. coli JM101 (pRMAB).

Diese Versuche sollten zur Klärung der Frage dienen, ob durch gleichzeitiges Vorhandensein von BADH und XMO die Aktivität, mit der Aldehyd aus Toluol und Pseudocumol gebildet wird, zunimmt oder abnimmt. Da einige Alkohol-Dehydrogenasen eine Reaktion katalysieren, deren Gleichgewicht beim physiologischen pH-Wert auf der-Seite des Alkohols liegt, könnte letztere Möglichkeit durchaus bestehen und folglich BADH die Aldehyd-Bildungsrate erniedrigen.

Tatsächlich konnten im Vergleich mit E. *coli* JM101 (pSPZ3) deutliche Unterschiede in der Biotransformationsaktivität festgestellt werden. Toluol wurde mit einer anfänglichen spezifischen Aktivität von 87 U/g CDW in seine entsprechenden Oxidationsprodukte umgewandelt. Die entsprechenden Aktivitäten für Benzylalkohol, Pseudocumol und 3,4-Dimethylbenzylalkohol betrugen 66, 73 und 42 U/g CDW. Wenn BADH vorhanden ist, bewirkt dies also anscheinend eine Erniedrigung der Biotransformationsaktivität von rekombinanten E. *coli* MJ101 als Biokatalysatoren.

Wenn Toluol zugegeben wurde, bildeten sich nacheinander Benzylalkohol, Benzaldehyd und Benzoesäure (Figur 3B). Im Zeitraum zwischen der 5. und 10. Minute wurde Benzaldehyd mit einer Aktivität von 34 U/g CDW gebildet, und die Säure wurde mit einer Aktivität von 1 U/g CDW im Zeitraum zwischen 10 und 40 Minuten gebildet. Nach etwa 20 Minuten begann die Konzentration an Benzylalkohol wieder anzusteigen (vermutlich wegen des Einsetzens der Rückreaktion). Nach 80 Minuten war nahezu kein Benzaldehyd mehr übrig und es wurde nur eine sehr geringe Menge an Benzoesäure gebildet.

Wenn Benzylalkohol als Substrat zugegeben wurde, ergaben sich sehr ähnliche Ergebnisse (nicht gezeigt). Wieder sank nach etwa 20 Minuten während der Bildung des Alkohols die Konzentration des Aldehyds. Somit verursacht die Einführung des Gens xylB eine beträchtliche Reduktion des Aldehyds zu Alkohol, was darauf hindeutet, dass die Bildung von Aldehyd durch zusätzliches BADH nicht gesteigert wird.

Wenn Pseudocumol als Substrat zugegeben wurde, wurde wieder eine nacheinander erfolgende Bildung von 3,4-Dimethylbenzylalkohol, 3,4-Dimethylbenzaldehyd und 3,4-Dimethylbenzoesäure festgestellt (Figur 4B). Im Zeitraum zwischen der 5. und 10. Minute wurde 3,4-Dimethylbenzaldehyd mit einer Aktivität von 15 U/g CDW gebildet. Die Säure bildete sich im Zeitraum zwischen 30 und 40 Minuten mit etwa der gleichen Aktivität (13 U/g CDW). Nach der raschen Bildung des Alkohols blieb die Aldehyd-Konzentration 20 Minuten lang relativ hoch. Trotzdem wurden am Ende der Reaktion der Alkohol und der Aldehyd vollständig in die Säure umgewandelt.

Wenn 3,4-Dimethylbenzylalkohol als Substrat zugegeben wurde, wurden 3,4-Dimethylbenzaldehyd und 3,4-Dimethylbenzoesäure gebildet (Figur 5B). Im Zeitraum zwischen 20 und 30 Minuten wurde die Säure mit einer spezifischen Aktivität von 8 U/g CDW gebildet. Die Aldehyd-Konzentration überstieg nie die Alkohol-Konzentration. Das längere Vorhandensein von 3,4-Dimethylbenzylalkohol zeigt, dass neben den Oxidationen wieder eine Reduktion von Aldehyd zu Alkohol stattfindet, für die BADH verantwortlich zu sein scheint.

### Beispiel 4: Wachstums-und Induktionskinetiken von E. coli JM101 (pSPZ3).

Für jeden Aktivitätspunkt wurde eine einzelne Kultur angezüchtet. Der Aktivitätsassay wurde wie oben beschrieben durchgeführt. Im Fall von Figur 5A wurde Pseudocumol (1,37 mM) zu einer Suspension ruhender E. coli JM101 (pSPZ3) (2,04-2,26 g 1⁻¹ CDW) in 50 mM Kaliumphosphat-Puffer, pH 7,4, der 1% (G/V) Glucose enthielt, gegeben. Die spezifischen Aktivitäten wurden mit Hilfe der gaschromatographisch bestimmten Mengen der in den ersten 5 Minuten der Umsetzung gebildeten Produkte berechnet. Der Pfeil zeigt an, zu welchem Zeitpunkt 0,1% (V/V) n-Octan zur Induktion der xylMA-Synthese zugegeben wurde. Die ausgefüllten Kreise bedeuten das Zelltrockengewicht (CDW) der uninduzierten Kulturen, die nicht ausgefüllten Kreise das Zelltrockengewicht der induzierten Kulturen und die Kreuze die spezifischen Aktivitäten der induzierten Kulturen.

Fig. 5(B) und (C) wurden wie Fig. 5(A) erhalten, mit dem Unterschied, daß die Zelldichte 2,26-2,44 g 1⁻¹ CDW betrug. Fig. 5(B) zeigt die Auswirkungen unterschiedlicher Mengen an n-Octan und Fif. 5(C) die von DCPK. Die Kreise geben das Zelltrockengewicht 3,5 Stunden nach der Induktion an und die Kreuze die spezifischen Aktivitäten der induzierten Kulturen.

Die XMO-Aktivität wurde nach der Induktion mit 0,1% (V/V) n-Octan (Fig. 5(A)) oder 0,05% (V/V) DCPK verfolgt. Die XMO-Aktivität wurde durch beide Verbindungen rasch induziert und erreichte nach 3 bis 3,5 Stunden Induktionszeit eine konstante Stärke von etwa 115 bzw. 105 U/g CDW im Falle von n-Octan bzw. DCPK. Im Vergleich mit nicht induzierten Zellen waren die Wachstumsraten der induzierten Zellen deutlich geringer.

Die Abhängigkeit der XMO-Aktivität von den Induktorkonzentrationen [im Bereich 0,00001- 1 % (V/V)] wurde bestimmt, indem E. coli JM101 (pSPZ3) bis zu einer Konzentration von 0,09 g CDW pro Liter angezogen und die Zellen mit unterschiedlichen Mengen an n-Octan und DCPK induziert wurden. Nach weiteren 3,5 Stunden Anzüchten wurden für jede Induktorkonzentration das Zelltrockengewicht und die XMO-Aktivität bestimmt (Fig. 5 (B) und (C)). Es ergaben sich sehr niedrige XMO-Aktivitäten, wenn weniger als 0,0001% (V/V) n-Octan oder 0,001% (V/V) DCPK in das Kulturmedium gegeben wurde. Maximale Induktion wurde bei DCPK-Konzentrationen von 0,005 bis 0,01% (V/V) und bei n-Octan-Konzentrationen zwischen 0,001 und 0,004% (V/V) festgestellt. Bei höheren Induktorkonzentrationen blieb die XMO-Aktivität konstant. Aufgrund des hohen Dampfdruckes von n-Octan wurden verschlossene Schüttelflaschen verwendet, wobei aber selbst dann nur ein Teil des n-Octans in dem wäßrigen Medium gelöst wird. Die Löslichkeit von n-Octan in destilliertem Wasser ist sehr niedrig und beträgt 0,7 mg/l oder 0,0001% (V/V).

In den Induktorkonzentrationsbereichen, in denen die Enzymaktivi-täten auf ihr Maximum anstiegen, nahmen die Zelldichten ab. Bei höheren Konzentrationen an n-Octan blieb die Enzymaktivität konstant. Höhere Konzentrationen an DCPK dagegen bewirkten eine weitere Abnahme der Zelldichten (Fig. 5 (B) und (C)). Außerdem wurde der direkte Einfluß von unterschiedlichen Induktorkonzentrationen auf das Wachstum von E. coli JM101 ohne Plasmid überprüft. Hohe Konzentrationen an n-Octan hatten keinen Einfluß auf das Zellwachstum. Im Gegensatz dazu hatten Konzentrationen an DCPK oberhalb 0,01% (V/V) eine Erniedrigung der Wachstumsrate zur Folge. Bei einer DCPK-Konzentration von 0,5% (V/V) wurde 3,5 Stunden nach der Induktion lediglich ein Zelltrockengewicht von 0,073 g/l bestimmt. Hohe DCPK-Konzentrationen üben offenbar eine toxische Wirkung auf die Zellen aus. Somit ist n-Octan der bessere Induktor.

Zusammengefaßt läßt sich folgendes feststellen. Bei der stufenweisen Oxidation von Toluol und Pseudocumol gibt es zwei signifikante Unterschiede. 3,4-Dimethylbenzylalkohol wird langsamer oxidiert als Benzylalkohol und 3,4-Dimethylbenzoesäure mit einer deutlich höheren Aktivität gebildet als Benzoesäure. Dies deutet darauf hin, dass die Variation von Substituenten auf die spezifischen Aktivitäten von XMO für oxidierte Substrate (Alkohole, Aldehyde) andere Auswirkungen zeigt als auf die spezifische Aktivität für unoxidierte Substrate, wie Toluol und Pseudocumol, für welche XMO sehr ähnliche Aktivitäten aufweist. Ein weiteres wichtiges Ergebnis ist, dass die Säuren solange nicht gebildet werden, bis das Toluol oder Pseudocumol mehr oder weniger vollständig verbraucht sind. Falls dies generell so sein sollte, hat XMO eindeutig eine höhere Affinität für Toluol und Pseudocumol als für die entsprechenden Aldehyde. Das Vorhandensein von BADH hat geringere Aktivitäten für die Produktbildung zur Folge und sogar die Rückbildung von Benzylalkohol. Die BADH enthaltenden Zellen akkumulieren die Aldehyde eindeutig langsamer. BADH scheint die Wirkung der E.-coli-Dehydrogenasen drastisch zu steigern, wobei das Gleichgewicht dieser Dehydrogenase-Reaktion auf der Seite des Alkohols zu liegen scheint. Dies wird durch thermodynamische Berechnungen nach im Stand der Technik bekannten Verfahren (26 bis 29) und durch enzymkinetische Untersuchungen (16-18) bestätigt.

### Literaturverzeichnis

1. Harayama, S., Kok, M. und Neidle, E.L. (1992), Annu. Rev. Microbiol. 46, 565-601
2. Harayama, S., Rekik, M., Wubbolts, M., Rose, K., Leppik, R.A. und Timmis, K.N. (1989), J. Bacteriol. 171(9), 5048-5055
3. Abril, M.-A., Michan., C., Timmis, K.N. und Ramos, J.L. (1989), J. Bacteriol. 171(12), 6782-6790
4. Williams, P.A., Shaw, L.M., Pitt, C.W. und Vrecl, M. (1997), Microbiology 143, 101-107
5. Ramos, J.L., Marqués, S. und Timmis, K.N. (1997), Ann. Rev. Microbiol. 51, 341-373
6. Suzuki, M., Hayakawa, T., Shaw, J.P., Rekik, M. und Harayama, S. (1991), J. Bacteriol. 173(5), 1690-1695
7. Shaw, J.P. und Harayama, S. (1992), Eur. J. Biochem. 209, 51-61
8. Wubbolts, M. (1994),Dissertation, Rijksuniversiteit Groningen
9. Shaw, J.P. und Harayama, S. (1995), J. Ferm. Bioeng. 79(3), 195-199
10. Baptist, J.N., Gholson, R.K. und Coon, M.J. (1963), Bioch. Biophys. Acta 69, 40-47
11. Chakrabarty, A.M., Chou, G. und Gunsalus, L.G. (1973), Proc. Natl. Acad. Sci. USA 70(4), 1137-1140
12. Kok, M., Oldenhuis, R., v.d. Linden, M.P.G., Raatjes, P., Kingma, J., v. Lelyveld, P.H. und witholt, B. (1989), J. Biol. Chem. 264(10), 5435-5441
13. van Beilen, J.B., Wubbolts, M.G. und Witholt, B. (1994), Biodegradation 5, 161-174
14. Shanklin, J., Whittle, E. und Fox, B.G. (1994), Biochemistry 33, 12787-12794
15. Shanklin, J. Achim, C., Schmidt, H., Fox, B.G. und Münck, E. (1997), Proc. Natl. Acad. Sci. USA 94, 2981-2986
16. Shaw, J.P. und Harayama, S. (1990), Eur. J. Biochem. 191, 5 705-714
17. Shaw, J.P., Schwager, F. und Harayama, S. (1992), Biochemical Journal 283, 789-794
18. Shaw, J.P., Rekik, M., Schwager, F. und Harayama, S. (1993), J. Biol. Chem. 268, 10842-10850
19. Kunz, D.A. und Chapman, P.J. (1981), J. Bacteriol. 146(1), 179-191
20. Wubbolts, M.G., Reuvekamp, P. und Witholt, B. (1994), Enzyme Microb. Technol. 16, 608-615
21. Harayama, S., Leppik, R.A., Rekik, M., Mermod, N., Lehrbach, 5 P.R., Reineke, W. und Timmis, K.N. (1986), J. Bacteriol. 167(2), 455-461
22. Grund, A., Shapiro, J., Fennewald, M., Bacha, P., Leahy, J., Markbreiter, K., Nieder, M. und Toepfer, M. (1975), J. Bacteriol. 123, 546-556
23. Yuste, L., Canosa, I. und Rojo, F. (1998), J. Bacteriol. 180(19), 5218-5226
24. Sambrook, J., Fritsch, E.F. und Maniatis, T. (1989), Molecular cloning: a laboratory manual, 2nd ed., Cold Spring Harbor Laboratory, Cold Spring Harbor, N.Y.
25. Panke, S., Witholt, B., Schmid, A. und Wubbolts, M.G. (1998), Appl. Environ. Microbiol. 64(6), 2032-2043
26. Mavrovouniotis, M.L. (1990), Biotechnol. Bioeng. 36, 1070-1082
27. Mavrovouniotis, M.L. (1991) The Journal of Biological Chemistry 266(22), 14440-14445
28. Dean, J.A. (Hrsg.) (1985), Lange's Handbook of Chemistry, 13. Auflage, McGraw-Hill Book Company
29. Leonardo, M.R., Dailly, Y. und Clark, D.P. (1996), J. Bacteriol. 178(20), 6013-6018
30. Hanahan, D. (1983), J. Mol. Biol. 166, 557-580
31. Panke, et al., (1999) Applied and Environmental Microbiology 65, 2324-2332
32. T.J. Silhavy, M.L. Berman und L.W. Enquist, Experiments with Gene Fusions, Cold Spring Harbor Laboratory, Cold Spring Harbor, NY (1984)
33. Ausubel, F.M. et al.,(1987) Current Protocols in Molecular Biology, Greene Publishing Assoc. and Wiley Interscience
34. Pouwels P. H. et al, (1985) "Cloning Vectors", Hrsg, Elsevier, Amsterdam-New York-Oxford
35. F. Ausubel et al.,(1997) Current Protocols in Molecular Biology, Wiley Interscience, New York

## Patentansprüche

1. Verfahren zur Herstellung aromatischer Aldehyde und/oder Carbonsäuren der allgemeinen Formel I
Ar-(CH₂)ₙ-R¹ (I)
worin
Ar für einen gegebenenfalls ein- oder mehrfach substituierten einkernigen aromatischen Ring steht;
R¹ für eine Sauerstoff-haltige Gruppe -CHO oder -COOH steht; und
n für eine ganzzahligen Wert von 0 bis 15 steht,
**dadurch gekennzeichnet, daß** man
a) in einem Kulturmedium, welches ein aromatisches Substrat der Formel II
Ar-R² (II)
worin
Ar die oben angegebenen Bedeutungen besitzt, und
R² für -CH=CB₂ oder -(CH₂)ₙ₊₁R³ steht, worin n wie oben angegeben definiert ist und R³ für H oder OH steht; oder, wenn R¹ für -COOH steht, R² auch für -(CH₂)ₙR⁴ stehen kann, worin n wie oben angegeben definiert ist und R⁴ für -CHO steht;
enthält, einen Mikroorganismus kultiviert, der Xylolmono- , Oxygenase (XMO) exprimiert wobei die exprimierte XMO die Oxidation des Substrats des Formel II zu eine Verbindung des Formel I katalysiert; und
b) die Verbindung(en) der Formel I aus dem Kulturmedium isoliert, wobei der XMO exprimierende Mikroorganismus im wesentlichen keine Benzylalkoholdehydrogenase (BADH) und gegebenenfalls keine Benzaldehyddehydrogenase (BZDH)-Aktivität besitzt.

2. Verfahren nach Anspruch 1, **dadurch gekennzeichnet, daß** man eine Verbindung der Formel I herstellt, worin R¹ für COOH steht.

3. Verfahren nach einem der vorherigen Ansprüche, **dadurch gekennzeichnet, daß** man einen rekombinanten Mikroorganismus verwendet, welcher mit einem Expressionsvektor transformiert ist, der unter der genetischen Kontrolle des regulativen alk-Systems aus Pseudomonas oleovorans GPol die für XMO kodierenden Gene xylM und xylA in operativer Verknüpfung enthält.

4. Verfahren nach Anspruch 3, **dadurch gekennzeichnet, daß** der Mikroorganismus mit dem Expressionsplasmid pSPZ3 transformiert ist.

5. Verfahren nach einem der vorhergehenden Ansprüche, **dadurch gekennzeichnet, daß** der Mikroorganismus ein Bakterium der Gattung Escherichia ist.

6. Verfahren nach einem der Ansprüche 3 bis 5, **dadurch gekennzeichnet, daß** man die Enzymexpression durch Zugabe eines Induktors zum Kulturmedium startet.

7. Verfahren nach einem der vorhergehenden Ansprüche, **dadurch gekennzeichnet, daß** man eine Verbindung der Formel II, worin R² für -CH=CH₂, -CH₃ oder -CH₂OH steht, mit einem Mikroorganismus umsetzt, welcher XMO-Aktivität exprimiert.

8. Verfahren nach Anspruch 3, **dadurch gekennzeichnet, daß** man Xylolmonoxygenase aus Pseudomonas putida mt-2 exprimiert.

9. Verwendung eines rekombinanten Mikroorganismus, enthaltend einen Expressionsvektor, der unter der genetischen Kontrolle des alk-Regulationssystems aus Pseudomonas oleovorans GPol die für XMO kodierenden Gene xylM und xylA in operativer Ver-5 knüpfung enthält, zur mikrobiologischen Herstellung aromatischer Verbindungen der allgemeinen Formel I.

10. Verwendung nach Anspruch 9, wobei der Mikroorganismus ausgewählt ist unter Bakterien der Gattung Escherichia und Pseudomonas.

11. Verwendung nach Anspruch 9 oder 10, wobei der Mikroorganismus transformiert ist mit dem Plasmid pSPZ3.

12. Verwendung eines Expressionskonstruktes, das unter der genetischen Kontrolle des regulativen alk-Systems aus Pseudomonas oleovorans GPol die für XMO kodierenden Gene xylM und xylA in operativer Verknüpfung enthält, zur mikrobiologischen Herstellung aromatischer Verbindungen der allgemeinen Formel I.

## Claims

1. A process for the preparation of aromatic aldehydes and/or carboxylic acids of the formula I
Ar-(CH₂)ₙ-R¹ (I)
where
Ar is an optionally mono- or polysubstituted mononuclear aromatic ring,
R¹ is an oxygen-containing group -CHO or -COOH, and
n is an integer from 0 to 15,
which comprises
a) culturing, in a culture medium comprising an aromatic substrate of the formula II
Ar-R² (II)
where
Ar is as defined above and
R² is -CH=CH₂ or -(CH₂)ₙ₊₁R³, where n is as defined above and R³ is H or OH; or, if R¹ is -COOH, R² can also be -(CH₂)ₙR⁴ where n is defined above and R⁴ is -CHO,
a microorganism which expresses xylene monooxygenase (XMO), wherein the expressed XMO catalyzes the oxidation of the substrate of formula II to a compound of formula I, and
b) isolating the compound(s) of the formula I from the culture medium, wherein the XMO-expressing microorganism has essentially no benzyl alcohol dehydrogenase (BADH) and optionally no benzaldehyde dehydrogenase (BZDH) activity.

2. The process according to claim 1, wherein a compound of formula I is produced in which R¹ is COOH.

3. The process according to any of the preceding claims, wherein a recombinant microorganism is used which has been transformed with an expression vector which comprises the XMO-encoding genes xylM and xylA in operable linkage under the genetic control of the alk regulatory system of Pseudomonas oleovorans GPol.

4. The process according to claim 3, wherein the microorganism has been transformed with the expression plasmid pSPZ3.

5. The process according to any of the preceding claims, wherein the microorganism is a bacterium of the genus Escherichia.

6. The process according to any of claims 3 to 5, wherein the enzyme expression is started by adding an inductor to the culture medium.

7. The process according to one of the preceding claims, wherein a compound of the formula II, where R² is -CH=CH₂, -CH₃, or -CH₂OH is converted using a microorganism which expresses XMO activity.

8. The process according to claim 3, wherein xylene monooxygenase of Pseudomonas putida mt-2 is expressed.

9. The use of a recombinant microorganism comprising an expression vector which comprises the XMO-encoding genes xylM and xylA in operable linkage under the genetic control of the alk regulatory system of Pseudomonas oleovorans GPo1, for the microbiological production of aromatic compounds of the formula I.

10. The use according to claim 9, the microorganism being selected from the bacteria of the genus Escherichia and Pseudomonas.

11. The use according to claim 9 or 10, the microorganism being transformed with the plasmid pSPZ3.

12. The use of an expression construct which comprises the XMO-encoding genes xylM and xylA in operable linkage and under the genetic control of the alk regulatory system of Pseudomonas oleovorans GPol, for the microbiological production of aromatic compounds of the formula I.

## Revendications

1. Procédé de préparation d'aldéhydes aromatiques et/ou d'acides carboxyliques de formule générale I
Ar- (CH₂)ₙ-R¹ (I)
dans laquelle :
Ar représente un cycle aromatique mononucléaire éventuellement substitué une ou plusieurs fois;
R¹ représente un groupe contenant de l'oxygène -CHO ou -COOH ; et
n est une valeur de nombre entier de 0 à 15,
**caractérisé en ce que** :
a) on cultive, dans un milieu de culture, lequel contient un substrat aromatique de formule II
Ar-R² (II)
dans laquelle :
Ar a les significations données ci-dessus, et
R² représente -CH=CH₂ ou -(CH₂)ₙ₊₁R³, où n est défini comme donné ci-dessus et R³ représente H ou OH;
ou, quand R¹ représente -COOH, R² peut également représenter - (CH₂)ₙR⁴, où n est défini comme donné ci-dessus et R⁴ représente -CHO;
un microorganisme qui exprime la xylolmonoxygénase (XMO), la XMO exprimée catalysant l'oxydation du substrat de formule II en un composé de formule I; et
b) on isole le/les composés de formule I du milieu de culture, le microorganisme exprimant la XMO ne présentant essentiellement aucune activité de benzylalcooldéshydrogénase (BADH) ni éventuellement de benzaldéhydedéshydrogénase (BZDH).

2. Procédé selon la revendication 1, **caractérisé en ce qu'**on prépare un composé de formule I, dans laquelle R¹ représente COOH.

3. Procédé selon l'une quelconque des revendications précédentes, **caractérisé en ce qu'**on utilise un microorganisme recombinant, lequel est transformé par un vecteur d'expression qui contient, en combinaison opérative, sous contrôle génétique du système ALK régulateur dérivé de Pseudomonas oleovorans GPol, les gènes xylM et xylA codant la XMO.

4. Procédé selon la revendication 3, **caractérisé en ce que** le microorganisme est transformé par le plasmide d'expression pSPZ3.

5. Procédé selon l'une quelconque des revendications précédentes, **caractérisé en ce que** le microorganisme est une bactérie de la souche Escherichia.

6. Procédé selon l'une quelconque des revendications 3 à 5, **caractérisé en ce qu'**on initie l'expression d'enzyme par l'addition d'un inducteur dans le milieu de culture.

7. Procédé selon l'une quelconque des revendications précédentes, **caractérisé en ce que** l'on fait réagir un composé de formule II, dans laquelle R² représente -CH=CH₂, -CH₃ ou -CH₂OH, avec un microorganisme, lequel exprime l'activité XMO.

8. Procédé selon la revendication 3, **caractérisé en ce qu'**on exprime la xylolmonoxygénase à partir de Pseudomonas putida mt-2.

9. Utilisation d'un microorganisme recombinant, contenant un vecteur d'expression qui contient, en combinaison opérative, sous contrôle génétique du système ALK régulateur derivé de Pseudomonas oleovorans GPol, les gènes xylM et xylA codant la XMO, pour la préparation microbiologique de composés aromatiques de formule générale I.

10. Utilisation selon la revendication 9, dans laquelle le microorganisme est sélectionné parmi les bactéries de la souche Escherichia et Pseudomonas.

11. Utilisation selon la revendication 9 ou 10, dans laquelle le microorganisme est transformé par le plasmide pSPZ3.

12. Utilisation d'une construction d'expression qui contient, en combinaison opérative, sous contrôle génétique du système ALK régulateur derivé de Pseudomonas oleovorans GPol, les gènes xylM et xylA codant le XMO, pour la préparation microbiologique de composés aromatiques de formule générale I.
